# DEMANDE DE BREVET EUROPEEN

(11) **EP 0 825 220 A1**
(43) Date de publication de la demande: **25.02.1998**
(21) Numéro de dépôt: 97401737.8
(22) Date de dépôt: 18.07.1997
(51) Int. Cl.: C08G 61/12, C08G 73/06, C07D 209/40, A61K 7/13, A61K 7/00, A61K 7/48

(54) **Produits dérivés de 2-imino-2,3-dihydro-1H-indoles, procédés de préparation, utilisation en cosmétique et compositions cosmétiques les mettant en oeuvre**

(30) Priorité: 23.08.1996 FR 9610411
(71) Demandeur: L'OREAL, 75008 Paris (FR)
(72) Inventeur: Lagrange, Alain, 77450 Coupvray (FR)
(74) Mandataire: Dodin, Catherine

(57) **Abrégé**

La présente demande concerne des produits dérivés de 2-imino-2,3-dihydro-1H-indoles résultant de la polymérisation oxydative d'au moins un composé de formule (I) ou (Il) suivantes : 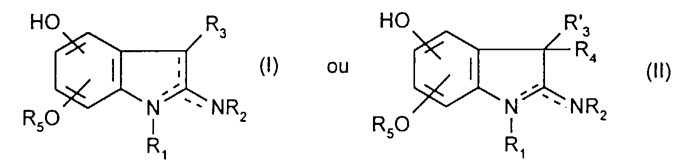 dans lesquelles :
- R₁, R₂ et R₃ désignent hydrogène, alkyle, carboxyle, alcoxycarbonyle, monohydroxyalkyle, polyhydroxyalkyle, alcoxyalkyle, monoalkylaminoalkyle ou dialkylaminoalkyle ;
- R'₃ et R₄ désignent alkyle, carboxyle, alcoxycarbonyle, monohydroxyalkyle, polyhydroxyalkyle, alcoxyalkyle, monoalkylaminoalkyle ou dialkylaminoalkyle ;
- R₅ désigne d'hydrogène, alkyle, monohydroxyalkyle, polyhydroxyalkyle, alcoxyalkyle, monoalkylaminoalkyle ou dialkylaminoalkyle ;
et leurs sels d'addition avec un acide, ainsi que leurs utilisations en cosmétique pour le maquillage des phanères et/ou de la peau.

## Description

La présente invention est relative à de nouveaux dérivés de 2-imino-2,3-dihydro-1H-indoles, leur procédé de préparation, leur utilisation en teinture des fibres kératiniques telles que les cheveux et les procédés de teinture les mettant en oeuvre.

L'utilisation de pigments colorés présente un grand intérêt dans le domaine cosmétique, notamment dans les produits destinés au maquillage des phanères et/ou de la peau.

On utilise généralement des pigments minéraux ou des pigments issus de colorants directs de synthèse ou de carbone pur dans le cas des pigments noirs. Ces différents produits présentent, suivant les applications, des problèmes de mise en oeuvre et ne sont pas toujours exempts de problèmes au niveau compatibilité et toxicologie.

La Demanderesse vient maintenant de découvrir de nouveaux produits utilisables comme pigments, en particulier en cosmétique qui sont des produits particulièrement intéressants au niveau des colorations qu'ils permettent d'obtenir ainsi que dans leur utilisation cosmétique.

Les produits conformes à l'invention sont obtenus par un processus de polymérisation oxydative mettant en oeuvre au moins un 2-imino-2,3-dihydro-1H-indole.

Par analogie et simplification, on appellera "produit indolique" ou "polymère indolique", le produit obtenu par polymérisation oxydative de différents composés comportant au moins un 2-imino-2,3-dihydro-1 H-indole.

La présente invention a donc pour objet de nouveaux produits indoliques tels que définis ci-après.
Un autre objet de l'invention est relatif à un produit sous forme de particules minérales ou organiques, comportant dans ou sur les particules, un produit indolique tels que défini ci-après.
Un autre objet de l'invention est constitué par le procédé de préparation de ces produits.
L'invention a également pour objet l'application cosmétique de ces produits indoliques, notamment dans les produits de maquillage de la peau et/ou des phanères et dans les produits destinés à la protection de l'épiderme humain contre les rayonnements UV.

Les produits indoliques conformes à l'invention résultent de la polymérisation oxydative d'au moins un composé répondant à la formule (I) ou à la formule (II) suivantes : 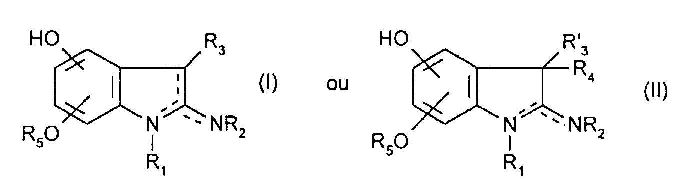 dans lesquelles :
- R₁, R₂ et R₃, identiques ou différents, désignent un atome d'hydrogène, un radical alkyle en C₁-C₄, carboxyle, alcoxy(C₁-C₄)carbonyle, monohydroxyalkyle en C₁-C₄, polyhydroxyalkyle en C₂-C₄, alcoxy(C₁-C₄) alkyle en C₁-C₄, monoalkyl(C₁-C₄) aminoalkyle en C₁-C₄ ou dialkyl(C₁-C₄) aminoalkyle en C₁-C₄ ;
- R'₃ et R₄, identiques ou différents, désignent un radical alkyle en C₁-C₄, carboxyle, alcoxy(C₁-C₄) carbonyle, hydroxyalkyle en C₁-C₄, polyhydroxyalkyle en C₂-C₄, alcoxy(C₁-C₄) alkyle en C₁-C₄, monoalkyl(C₁-C₄) aminoalkyle en C₁-C₄ ou dialkyl(C₁-C₄) aminoalkyle en C₁-C₄ ;
- R₅ désigne un atome d'hydrogène, un radical alkyle en C₁-C₄, monohydroxyalkyle en C₁-C₄, polyhydroxyalkyle en C₂-C₄, alcoxy(C₁-C₄) alkyle en C₁-C₄, mo-noalkyl(C₁-C₄) aminoalkyle en C₁-C₄ ou dialkyl(C₁-C₄) aminoalkyle en C₁-C₄ ;
lesdits radicaux alkyle ou alcoxy pouvant être linéaires ou ramifiés.

Les composés de formules (I) et (II) peuvent également se présenter sous la forme d'un sel d'addition avec un acide, et notamment sous la forme de chlorhydrates, de bromhydrates, de sulfates, de tartrates, de lactates ou d'acétates.

Chacune des formules (I) et (II) définies ci-dessus peut donner lieu à plusieurs formes tautomères dont la prépondérance et/ou la stabilité de chaque forme tautomère dépendra de la nature des différents substituants R₁, R₂, R₃, R'₃ et R₄.

La formule (I) peut donner lieu aux 3 formes tautomères suivantes :

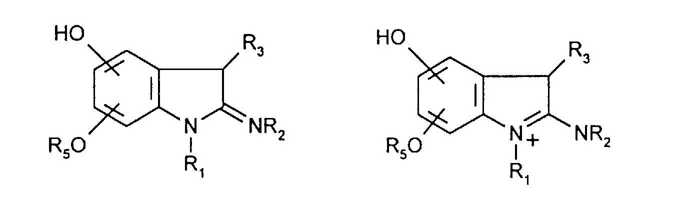

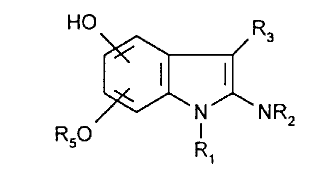

La formule (II) peut donner lieu aux deux formes tautomères suivantes :

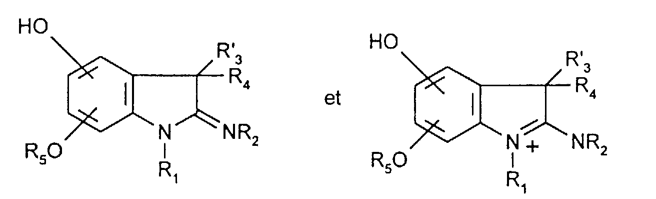

Parmi les composés de formules (I) et (II) préférentiels et leurs sels d'addition avec un acide, on peut citer plus particulièrement le 5,6-dihydroxy-1,3-dihydro-indol-2-ylidène-amine et ses sels d'addition avec un acide.

Les composés particuliers de formule (IA) ou (IIA) suivantes, ou leurs autres formes tautomères : 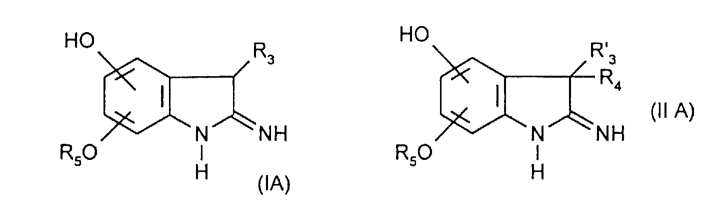 dans lesquelles R₃, R'₃, R₄ et R₅ ont les mêmes significations que celles indiquées ci-dessus dans les définitions des formules (I) et (II), peuvent être obtenus selon un procédé décrit dans le brevet RG Glushkov USSR Patent 179 320 (1965) et dans le document Chem. Abstr. 65 , 2225 (1966), et correspondant aux schémas A et A' suivants :

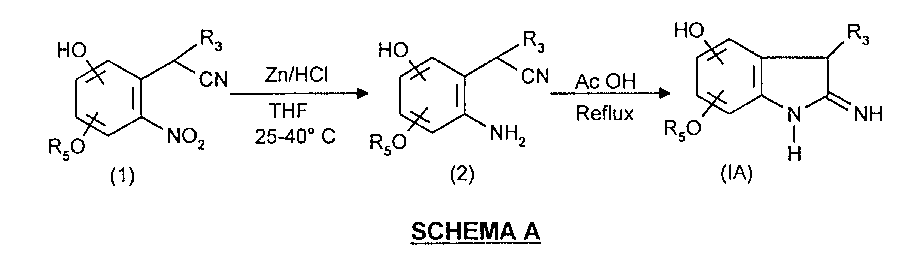

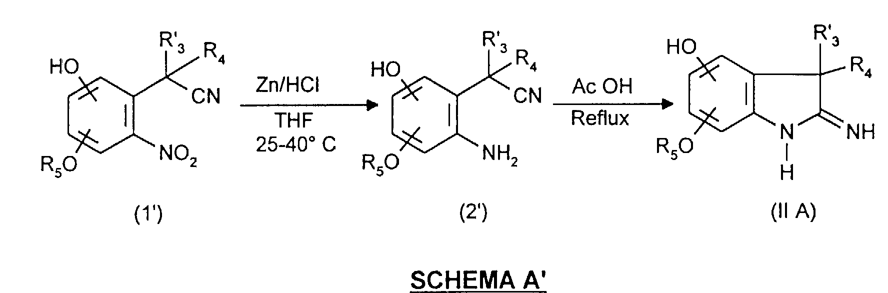

Dans les schémas A et A' définis ci-dessus, les significations des radicaux R₃, R'₃, R₄ et R₅ des formules (1), (1'), (2) et (2') sont identiques à celles indiquées précédemment dans les formules (I) et (II).

Il s'agit d'un procédé en deux étapes à partir des composés de départ de formule (1) ou (1') ayant pour structure celle d'ortho-nitro phénylacétonitriles dont la méthode de synthèse est connue dans la littérature (M. MAKOSZA, J. WINIARSKI, Acc. Chem. Res., 87, 1987, 282 ; M. MAKOSZA, W. DANIKIEWICZ, K. WOJCIECHOWSKI; Lilbrgs, Ann. Chem. 1988, 203.

La première étape est soit une réduction chimique en présence d'un solvant organique à l'aide de métaux comme le zinc ou l'étain, soit une hydrogénation sélective à l'aide d'un catalyseur comme le palladium ou le platine. Les solvants utilisés sont de préférence des éthers et plus particulièrement le tetrahydrofurane (THF). La température de réaction est de préférence comprise entre 25° C et la température de reflux du solvant et plus particulièrement entre 25 et 40° C.

La seconde étape est une réaction de cyclisation en milieu acide, en présence d'un solvant organique. On utilise de préférence l'acide acétique. La température de réaction est celle de reflux du solvant. Le produit final de formule (lA) ou (IIA) est isolé, de préférence sous la forme d'un sel d'addition d'acide. On l'obtient par précipitation du milieu réactionnel en milieu acide. Par exemple, pour obtenir un chlorhydrate, on fait passer un courant de HCI gazeux en fin de réaction.

Les composés de formule (IB) ou (IIB) particuliers, correspondant respectivement aux composés de formule (I) et de formule (II) dans lesquelles R₁ est un atome d'hydrogène et les composés de formule (IC) ou (IIC) particuliers correspondant respectivement aux composés de formule (I) et de formule (II) dans lesquelles R₁ et R₂ sont tous les deux différents d'un atome d'hydrogène, peuvent être obtenus selon un procédé de préparation, faisant référence à la littérature, et répondant aux schémas B et B' suivants :

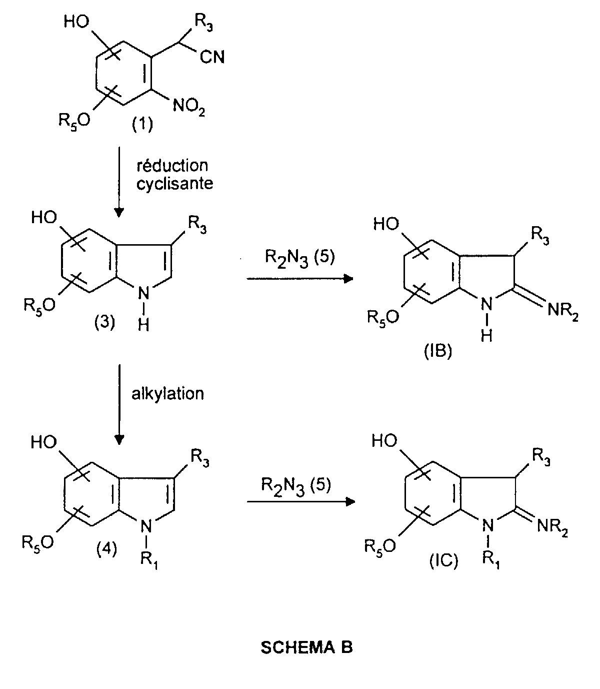

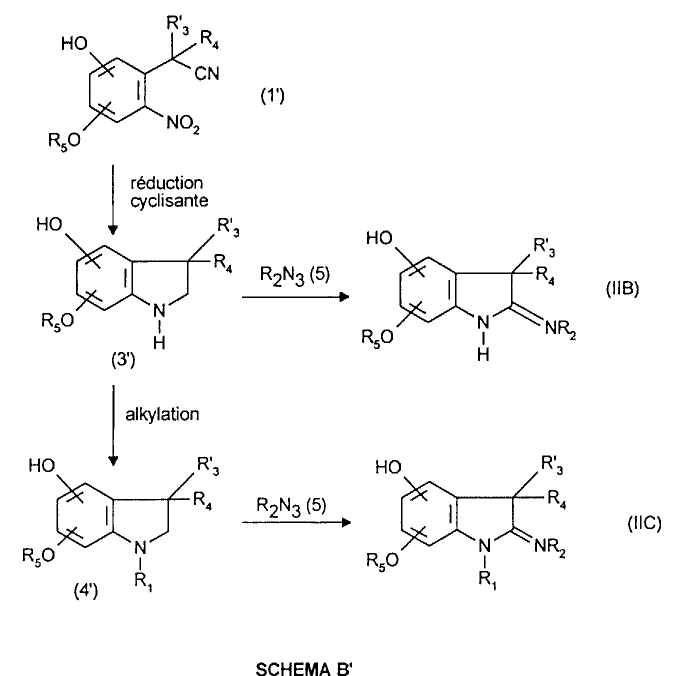

Le composé (1) ou (1') peut être traité dans des conditions de réduction cyclisante selon des méthodes connues, comme par exemple celle décrite par MAKOSZA M. & coll. dans Liebigs Ann. Chem., 203, (1988) pour conduire respectivement à l'indole (3) ou (3'). L'indole (3) ou (3') peut être alkylé pour conduire respectivement à l'indole (4) ou (4') selon des méthodes classiques décrites dans *"Heterocyclic compounds : Indoles"* part II p. 72-73, édité par N.J. HOULIAN, Wiley-interscience.

Les composés (3) ou (3') et (4) ou (4') peuvent réagir avec un acide de structure (5) pour conduire respectivement aux 2-iminoindolines (IB) ou (IIB) et (IC) ou (IIC) selon une méthode déjà décrite [HARMON R.E. & coll., J. Org. Chem. 38(1), 11, (1973)].

Les composés de structures (IB) ou (IIB) et (IC) ou (IIC) peuvent également être obtenus par réaction d'une amine R₂NH₂ respectivement sur un dérivé 2-indolinethione de structure (6) ou (6') et (7) ou (7') comme décrit par HINO T. & coll. dans Tetrahedron 27, 775, (1971) et représenté dans les schémas C et C' suivants :

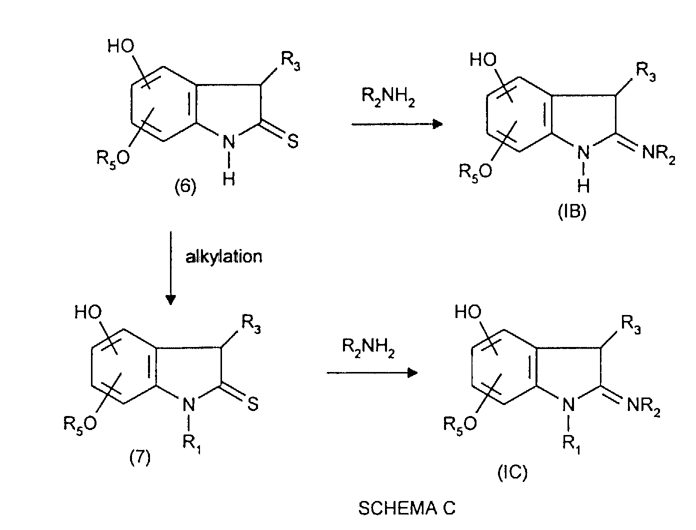

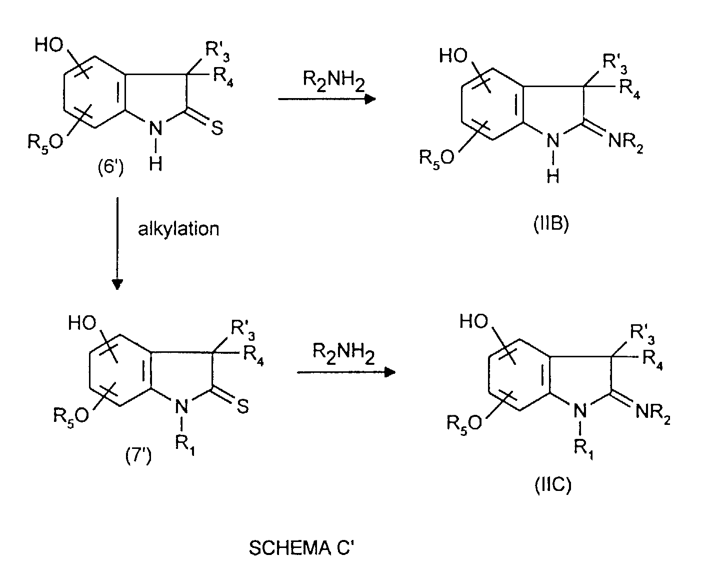

Les produits indoliques dérivés de 2-imino-2,3-dihydro-1H-indoles conformes à l'invention peuvent également être obtenus par cooxydation d'au moins un composé de formule (I) ou (II) définies ci-dessus avec au moins un dérivé indolique et/ou avec au moins un dérivé indolinique.

A titre de dérivé indolique, on peut plus particulièrement citer les composés répondant à la formule (III) suivante : 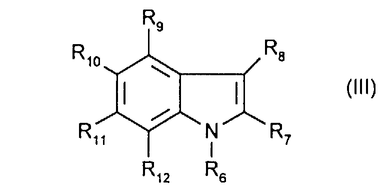 dans laquelle :
- R₆ et R₈, identiques ou différents, représentent un atome d'hydrogène ou un radical alkyle en C₁-C₄ ;
- R₇ représente un atome d'hydrogène, un radical alkyke en C₁-C₄, carboxyle ou alcoxy(C₁-C₄) carbonyle;
- R₉ et R₁₂, identiques ou différents, représentent un atome d'hydrogène, un radical hydroxyle, alkyle en C₁-C₄, amino, alcoxy en C₁-C₄, acyl(C₂-C₄)oxy ou acyl(C₂-C₄)amino ;
- R₁₀ représente un atome d'hydrogène ou d'halogène, un radical hydroxyle, alkyke en C₁-C₄, alcoxy en C₁-C₄, amino, acyl(C₂-C₄)oxy, acyl(C₂-C₄)amino ou triméthylsilyloxy ;
- R₁₁ désigne hydrogène, un groupement hydroxy, alcoxy(C₁-C₄), amino acyl(C₂-C₄)oxy, acyl(C₂-C₄)amine, triméthylsiloxy, hydroxyalkyl(C₂-C₄)amino ;
- R₁₀ et R₁₁ peuvent former conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy; au moins l'un des groupements R₉ à R₁₂ représente un groupement OZ ou NHR, un seul au plus des groupements R₉ à R₁₂ désignant NHR ; et au plus deux des groupements R₉ à R₁₂ désignent OZ, dans le cas ou Z désigne hydrogène, ces groupements sont en position 5 et 6 ; et au moins un des groupements R₉ à R₁₂ représente hydrogène, dans le cas où un seul de ces groupements désigne hydrogène, un seul groupement parmi R₉ à R₁₂ désigne alors NHR ou OZ, et les autres groupements désignent alkyle en C₁-C₄ ; R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄ triméthylsilyle et les sels correspondants.

Les composés indoliques de formule (III) sont choisis, en particulier, parmi le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole.

Le 5,6-dihydroxyindole, le 6-hydroxyindole, le 3-méthyl 5,6-dihydroxyindole, le 2-méthyl 5,6-dihydroxyindole, le 1-méthyl 5,6-dihydroxyindole, le 2-carboxy 5,6-dihydroxyindole, utilisés seuls ou en mélange sont préférés.

Les dérivés d'indoline que l'on utilise en association avec les composés de formule (I) ou (II) pour obtenir les produits conformes à l'invention sont choisis en particulier parmi les composés de formule (IV) suivante : 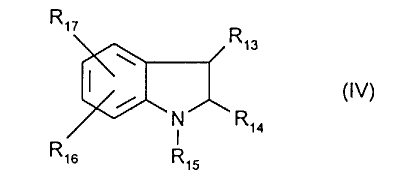 dans laquelle :
- R₁₃ et R₁₅ représentent, indépendamment l'un de l'autre, un atome d'hydrogène ou un groupement alkyle en C₁-C₄ ;
- R₁₄ représente un atome d'hydrogène, un groupement alkyle en C₁-C₄, un groupement carboxyle ou alcoxy (C₁-C₄) carbonyle ;
- R₁₆ désigne un atome d'hydrogène ou d'halogène, un groupement alkyle en C₁-C₄, hydroxyle, alcoxy (C₁-C₄), amino ou alkylamino en C₁-C₁₀ ;
- R₁₇ désigne un atome d'hydrogène, un groupement hydroxyle, alcoxy en C₁-C₄ ou amino;
avec la condition qu'au moins un des radicaux R₄ ou R₅ désigne un groupement hydroxyle, alcoxy ou amino ; et sous réserve que lorsque R₅ désigne un groupement amino, R₄ ne peut désigner un radical alkylamino ; R₁₆ et R₁₇ peuvent également former un cycle alkylènedioxy en C₁-C₂ et sont en position 5 et 6 ; ainsi que les sels correspondants.

Les sels sont des sels cosmétiquement acceptables, en particulier des chlorhydrates, bromhydrates, sulfates, méthanesulfonates. Les bromhydrates des composés ci-dessus sont particulièrement préférés.

Dans les composés de formule (IV), les radicaux alkyle en C₁-C₄ désignent de préférence méthyle, éthyle, propyle, isopropyle, butyle, isobutyle. Pour les radicaux alkyle en C₁-C₁₀, le radical alkyle en C₁-C₁₀, désigne de préférence méthyle, éthyle, propyle, isopropyle, butyle, isobutyle, 1-méthylhexyle, 1-méthylheptyle, 1-méthyloctyle, les radicaux alcoxy désignent de préférence méthoxy, éthoxy, propoxy et butoxy, halogène désigne brome, chlore ou iode.

Parmi les composés répondant à la formule (IV), les composés préférés utilisés conformément à l'invention, sont choisis parmi la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6 aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 6-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl) aminoindo-line, la 5,6-diméthoxyindoline et la 5,6-dihydroxy 2-carboxyindoline.

Lorsque les produits dérivés de 2-imino-2,3-dihydro-1H-indoles conformes à l'invention peuvent être préparés par cooxydation, on peut, de préférence, utiliser jusqu'à 50% en mole de dérivé indolique et/ou de dérivé indolinique par rapport au nombre total de moles de dérivés de formule (I) ou (II) à oxyder.

Les produits dérivés de 2-imino-2,3-dihydro-1H-indoles conformes à l'invention, peuvent être préparés selon différents processus de polymérisation oxydative.

Selon un premier type de processus, on procède à une simple oxydation à l'air. Dans ce cas, on n'utilise pas d'autre agent oxydant que l'oxygène de l'air et on opère de préférence à un pH alcalin dans un milieu eau ou eau/solvant.

L'oxydation à l'air peut également s'effectuer en présence d'un agent alcalin et/ou d'un catalyseur métallique d'oxydation tel que l'ion cuivrique.

Selon un deuxième type de procédé, la préparation des produits conformes à l'invention peut s'effectuer en présence d'un agent oxydant tel que le peroxyde d'hydrogène, les peracides et les persels.

On peut citer parmi les peracides et les persels, l'acide périodique et ses sels hydrosolubles, les permanganates et les bichromates, tels que de sodium ou de potassium, le persulfate d'ammonium et les peracides organiques.

Le sel d'acide périodique préféré est le periodate de sodium.

D'autres agents oxydants sont choisis parmi les chlorites alcalins, l'oxyde d'argent, le chlorure ferrique, l'oxyde de plomb, le nitrite de sodium ; les sels de terres rares tels que notamment le sel de cérium.

On peut également utiliser des oxydants organiques choisis parmi les ortho et parabenzoquinones, les ortho et parabenzoquinones monoimines et diimines, les 1,2- et 1,4-naphtoquinones, les 1,2 et les 1,4-naphtoquinones mono- ou diimines.

L'oxydation peut enfin être effectuée par utilisation d'iodure tel qu'un iodure de métal alcalin, alcalino-terreux ou d'ammonium en présence de peroxyde d'hydrogène.

Ces agents oxydants peuvent être activés éventuellement par un agent modificateur de pH et/ou par un catalyseur métallique d'oxydation.

L'oxydation est généralement réalisée dans une gamme de températures allant de la température ambiante à 100°C avec une préférence pour les températures comprises entre 20 et 80°C.

Il est généralement possible de procéder à la formation des produits indoliniques conformes à l'invention par oxydation par voie enzymatique. Cette oxydation s'effectue dans un milieu oxydant et en présence d'une enzyme à activité oxydante ou peroxydante telle que les enzymes choisies parmi la peroxydase de raifort, la chloroperoxydase, la peroxydase du lait, la cytochrome C-peroxydase, ainsi que des produits ayant une activité similaire, celle des enzymes peroxydantes telle que l'hémoglobine, la methémoglobine, la myoglobine, la metmyoglobine.

Pour les produits destinés à une application cosmétique, on utilise de préférence comme agents oxydants, le peroxyde d'hydrogène, l'acide périodique et ses sels, le permanganate de potassium, l'hypochlorite de sodium, le persulfate d'ammonium, le nitrite de sodium et le système iodure/peroxyde d'hydrogène. Lorsqu'on utilise un iodure en présence d'eau oxygénée, il s'agit préférentiellement d'iodure de sodium ou de potassium à une concentration pondérale comprise entre 1 et 6% par rapport au poids du milieu réactionnel.

L'ordre d'addition des composés intervenant dans la préparation du produit conforme à l'invention a peu d'importance à la condition que l'on incorpore en dernier lieu l'agent oxydant quand celui-ci est utilisé sans agent modificateur de pH et dans le cas du système oxydant iodure/peroxyde d'hydrogène, on introduit en dernier lieu, soit le peroxyde d'hydrogène, soit l'iodure.

Dans le cas où on utilise un agent modificateur de pH pour activer l'agent oxydant, on préfère ajouter en dernier lieu, soit l'agent oxydant, soit le modificateur de pH.
Les agents modificateurs de pH sont des agents acidifiants ou alcalinisants habituellement utilisés en cosmétique.

Avant l'utilisation de l'oxydant, le dérivé à oxyder est mis en solution aqueuse ou dans un milieu eau/solvant avec une proportion de solvant comprise entre 0,5 et 95% ou dans un milieu solvant pur.

Les solvants sont de préférence choisis parmi les alcools inférieurs en C₁-C₄, tels que l'alcool éthylique, l'alcool propylique ou isopropylique, l'alcool tertiobu-tylique, les alkylèneglycols tels que l'éthylèneglycol, le propylèneglycol, les al-kyléthers d'alkylèneglycols tels que les éthers monométhylique, monoéthylique et monobutylique de l'éthylèneglycol, les monométhyléthers de propylèneglycol et du dipropylèneglycol et le lactate de méthyle.

Ces solvants doivent par ailleurs pouvoir solubiliser le composé de formule (I) ou (II) et éventuellement le dérivé indolinique et/ou le dérivé indolique mis en oeuvre pour former le produit final conforme à l'invention. Le solvant préféré est l'éthanol et le milieu préférentiel d'oxydation est hydroalcoolique avec une teneur en éthanol comprise entre 1 et 15%.
Dans le procédé conforme à l'invention, les dérivés à oxyder représentent en général de 0,1 à 30% et de préférence de 1 à 20% en poids du poids total du milieu réactionnel.

Les temps de contact entre les dérivés à oxyder et les réactifs oxydant peuvent varier de quelques minutes à quelques jours suivant les procédés.

Les agents alcalins préférés sont la soude, les carbonates alcalins ou l'ammoniaque. Lorsqu'ils sont utilisés, leur concentration dans le milieu d'oxydation est comprise entre 5,10⁻⁴ et 10% en poids.

Pour préparer les produits conformes à l'invention, on utilise de préférence le procédé d'oxydation par l'eau oxygénée en présence d'ammoniaque.

Lorsque le processus d'oxydation est terminé, le produit indolinique coloré ainsi formé est isolé par filtration, centrifugation ou lyophilisation. Pour éliminer les traces de dérivés à oxyder n'ayant pas réagi, on rince le produit abondamment à l'eau avant ou après filtration ou centrifugation.

Dans le cas où l'on prépare le produit conforme à l'invention par simple oxydation à l'air, on préfère isoler le produit indolinique par lyophilisation.

Afin d'obtenir un produit homogène et de granulométrie suffisamment fine, il est ensuite possible de traiter le produit obtenu par des systèmes de broyages classiques par voie sèche ou humide. On peut également utiliser un procédé de micronisation.

La granulométrie du produit final est, de préférence, telle que le diamètre moyen des particules soit inférieur à 50 microns et de préférence inférieur à 20 microns.

De même, 90% des particules ont un diamètre généralement inférieur à 100 microns et de préférence à 50 microns.
Les produits dérivés de 2-imino-2,3-dihydro-1H-indoles conformes à l'invention, sont essentiellement des polymères généralement insolubles dans les milieux cosmétiques habituellement utilisés. Ces produits peuvent être cependant mis en oeuvre en solution dans des milieux solvants particuliers, par exemple en utilisant un milieu dont le pH est élevé.

Les produits dérivés de 2-imino-2,3-dihydro-1H-indoles conformes à l'invention peuvent selon un mode particulier de l'invention, être mis en oeuvre sous la forme d'une poudre constituée de particules minérales ou organiques, dont la dimension la plus grande est inférieure à 20 microns et comportant dans et/ou sur les particules les produits conformes à l'invention formés in situ par polymérisation oxydative d'au moins un composé de formule (I) ou (II) telle que définie ci-dessus.

Les poudres comportant les produits dérivés de 2-imino-2,3-dihydro-1H-indoles conformes à l'invention constituent un autre objet de l'invention.

Les particules minérales ou organiques contenues dans les poudres pigmentaires conformes à l'invention sont celles utilisées dans les poudres pigmentaires décrites dans la demande EP-A-O 575 605.

Les poudres conformes à l'invention sont préparées selon les différents types de procédé décrits dans la demande EP-A-0 575 605.

Un autre objet de l'invention consiste en l'utilisation de ces poudres dans et pour la préparation de compositions cosmétiques ou dermatologiques notamment dans tous les produits de traitement ou de soin de la peau et/ou des phanères. On appelle "phanères", les cheveux, les poils, les cils, les sourcils, les ongles.

Les produits indoliques conformes à l'invention selon un objet de l'invention, peuvent être utilisés en cosmétiques ou en dermatologie, notamment dans tous les produits de traitement ou de soin de la peau et/ou des phanères.
Les produits indoliques conformes à l'invention, dans leur application cosmétique, sont utilisés sous forme libre ou bien incorporés dans des poudres à base de particules minérales ou organiques, dans des compositions cosmétiques contenant dans un milieu cosmétiquement acceptable, à une concentration comprise de préférence entre 0,1 et 35% en poids et en particulier entre 0,5 et 20 % en poids par rapport au poids total de la composition.

Ces compositions peuvent être utilisées comme produits de maquillage, notamment des cils, des sourcils, de la peau, des ongles, tels que sous forme de fard à paupières, fards à joues, ligneurs encore appelés "eye-liners", mascaras pour les cils et les sourcils, les vernis à ongles, comme compositions tinctoriales pour cheveux, notamment pour réaliser une teinture temporaire des cheveux ou maquillage.

Ces compositions peuvent également être utilisées pour la protection de l'épiderme humain contre les rayonnements UV.

Les compositions peuvent se présenter sous forme de lotion, de lotion épaissie, de gel, de crème, de lait, de poudre, de stick et éventuellement être conditionnées en aérosol et se présenter sous forme de mousse ou de spray.

Lorsque les compositions sont utilisées pour le maquillage de la peau, des cils et des sourcils, elles peuvent notamment se présenter sous forme solide ou pâteuse, anhydre ou aqueuse, comme des émulsions huile-dans-eau ou eau-dans-huile ou encore des suspensions.

Ces compositions présentent l'avantage d'être particulièrement stables et de présenter une bonne innocuité.

Lorsque les compositions sont utilisées pour la protection de l'épiderme humain contre les rayonnements UV, elles constituent des compositions dites "solaires" et elles peuvent se présenter sous forme de suspensions ou de dispersions dans des solvants ou des corps gras, ou encore sous forme d'émulsions telles que crèmes et laits, de pommades, de gels, de bâtonnets solides ou de mousses aérosols.

Lorsqu'elles sont utilisées sous forme d'émulsions, elles peuvent contenir en outre des agents tensioactifs bien connus dans l'état de la technique, tels que des agents tensioactifs anioniques, non-ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousse, des agents hydratants, des parfums, des conservateurs, des agents antioxydants, des charges, des séquestrants, des agents de traitement tels que des polymères anioniques, cationiques, non ioniques, amphotères, ou leurs mélanges, des propulseurs, des agents alcalinisants ou acidifiants.

Les corps gras peuvent être constitués par une huile ou une cire ou leur mélange, les acides gras, les alcools gras, la vaseline, paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies parmi les cires animales, fossiles, végétales, minérales ou de synthèse. On peut citer en particulier les cires d'abeilles, les cires de Carnauba, de Candellila, de canne à sucre, du Japon, les ozokérites, la cire de Montan, les cires microcristallines, les paraffines.

Les compositions conformes à l'invention peuvent également contenir en plus des produits indoliniques tels que définis ci-dessus, des pigments généralement utilisés en cosmétique, notamment des pigments nacrés et/ou nacrants permettant de varier les colorations susceptibles d'être obtenues, ou d'augmenter la protection vis-à-vis du rayonnement ultraviolet. On utilise, dans ce dernier cas, plus particulièrement des pigments ou nanopigments d'oxydes métalliques tels que les oxydes de titane, de zinc, de cérium ou de zirconium.

Les nanopigments qui sont utilisés de façon préférentielle, sont des pigments ayant un diamètre moyen inférieur à 100 nm et de préférence compris entre 5 et 50 nm. Ils peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans COSMETICS and TOILETRIES, Février 1990, Vol. 105 pages 53-64, tels que des aminoacides, de la cire d'abeille, des acides gras, des alcools gras, des tensioactifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Lorsqu'elles sont utilisées pour la coloration temporaire des cheveux, elles se présentent sous forme de lotions plus ou moins épaissies, de gel, de mousse ou de spray contenant le produit indolinique dans un milieu aqueux ou eau/solvant(s) dans les proportions indiquées ci-dessus.

Lorsqu'elles sont utilisées pour le traitement des ongles, le produit indolinique est introduit dans un milieu pour vernis à ongles comprenant un solvant volatile et des polymères.

Un autre objet de l'invention est constitué par le procédé de coloration temporaire des cheveux, de maquillage de la peau et des phanères, de protection de l'épiderme humain contre les effets néfastes des rayonnements UV, mettant en oeuvre les produits indoliniques selon l'invention.
Ces produits peuvent être appliqués directement sous forme de poudre ou au moyen de compositions cosmétiques telles que définies ci-dessus.

Les exemples suivants sont destinés à illustrer l'invention sans pour autant présenter un caractère limitatif.

### EXEMPLES DE PREPARATION

### EXEMPLE 1

On a solubilisé 6,62 g (0,033 mole) de chlorhydrate de 5,6-dihydroxy-1,3-dihydroindol-2-ylideneamine dans 50 ml de solution aqueuse à 0,1 % d'ammoniaque. On a porté cette solution à 80°C et on a ajouté 3 ml d'une solution aqueuse d'ammoniaque à 20 % (0,033 mole). On a additionné à ce mélange 7,65 g d'eau oxygénée contenant 0,675 mole de peroxyde d'hydrogène en maintenant la température entre 80 et 85°C. L'addition terminée, on a maintenu la température à 80 C pendant 2,5 heures puis on a refroidi le milieu réactionnel. On a centrifugé le produit à 500 tours/minute pendant 10 minutes et repris avec 250 ml d'eau ; puis centrifugé à 500 tours/minutes pendant 10 minutes, cette opération a été répétée trois fois. On a obtenu après séchage 3,1 g de poudre brun foncé. Cette poudre a ensuite pu être micronisée selon les procédés classiques de micronisation. Dans une variante de ce procédé, le produit avant séchage a été passé en milieu humide dans un broyeur à billes.

### EXEMPLE 2

On a solubilisé 5;958 g (0,0297 mole) de chlorhydrate de 5,6-dihydroxy-1,3-dihydro-indol-2-ylideneamine et 0,492 g (0,0033 mole) de 5,6-dihydroxyindole dans 50 ml de solution aqueuse à 0,1% d'ammoniaque. On a porté ce mélange à 80°C puis on a ajouté 2,7 ml d'une solution aqueuse d'ammoniaque à 20% (0,0297 mole). On a additionné à ce mélange en 2 h 30 minutes, 28,8 g d'eau oxygénée contenant 0,033 mole de peroxyde d'hydrogène en maintenant la température entre 80° et 85°C. L'addition terminée, on a maintenu la température à 80°C pendant 3 heures puis on a refroidi le milieu réactionnel. On a essoré le produit noir et on l'a lavé à l'eau. On a obtenu après séchage 4,6 g de poudre noire qui a pu, comme à l'exemple 1, être micronisée. Le produit noir obtenu avant séchage pouvait, comme à l'exemple 1, être passé en milieu humide dans un broyeur à billes.

### EXEMPLE DE FORMULATION

### EXEMPLE A

On a préparé un mascara anhydre de composition suivante :
- Cire de Carnauba 5,0 g
- Cire de Candellila 5,0 g
- Ethanol 3,0 g
- Montmorillonite modifiée par une substance organique 4,0 g
- Lanoline 2,0 g
- Talc 10,0 g
- Poudre noire de l'exemple 1 2,0 g
- Isoparaffine qsp 100 g

Ce mascara waterproof est de couleur noire.

## Revendications

1. Produit indolique dérivé de 2-imino-2,3-dihydro-1 H-indoles, caractérisé par le fait qu'il résulte de la polymérisation oxydative d'au moins un composé de formule (I) ou (II) suivantes : 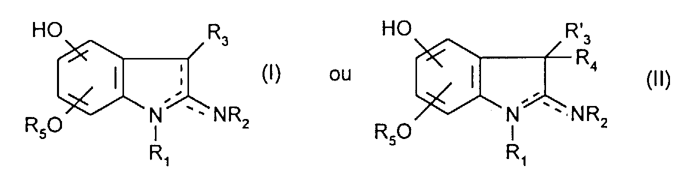 dans lesquelles :
- R₁, R₂ et R₃, identiques ou différents, désignent un atome d'hydrogène, un radical alkyle en C₁-C₄, carboxyle, alcoxy(C₁-C₄)carbonyle, monohydroxyalkyle en C₁-C₄, polyhydroxyalkyle en C₂-C₄, alcoxy(C₁-C₄) alkyle en C₁-C₄, monoalkyl(C₁-C₄) aminoalkyle en C₁-C₄ ou dialkyl(C₁-C₄) aminoalkyle en C₁-C₄ ;
- R'₃ et R₄, identiques ou différents, désignent un radical alkyle en C₁-C₄, carboxyle, alcoxy(C₁-C₄) carbonyle, monohydroxyalkyle en C₁-C₄, polyhydroxyalkyle en C₂-C₄, alcoxy(C₁-C₄) alkyle en C₁-C₄, monoalkyl(C₁-C₄) aminoalkyle en C₁-C₄ ou dialkyl(C₁-C₄) aminoalkyle en C₁-C₄ ;
- R₅ désigne un atome d'hydrogène, un radical alkyle en C₁-C₄, monohydroxyalkyle en C₁-C₄, polyhydroxyalkyle en C₂-C₄, alcoxy(C₁-C₄) alkyle en C₁-C₄, monoalkyl(C₁-C₄) aminoalkyle en C₁-C₄ ou dialkyl(C₁-C₄) aminoalkyle en C₁-C₄ ;
lesdits radicaux alkyle ou alcoxy pouvant être linéaires ou ramifiés, les composés de formules (I) et (II) pouvant se présenter sous la forme d'un sel d'addition avec un acide.

2. Produit indolique selon la revendication 1, caractérisé par le fait que les sels d'addition avec un acide sont choisis dans le groupe constitué par les chlorhydrates, les bromhydrates, les sulfates, les tartrates, les lactates et les acétates.

3. Produit indolique résultant de la polymérisation oxydative du 5,6-dihydroxy-1,3-dihydro-indol-2-ylidène-amine ou d'au moins l'un de ses sels d'addition avec un acide.

4. Produit indolique selon l'une quelconque des revendications 1 à 3 caractérisé par le fait qu'il résulte de la cooxydation d'au moins un composé de formule (I) ou (II) avec au moins un dérivé indolique et/ou avec au moins un dérivé indolinique.

5. Produit indolique selon la revendication 4, caractérisé par le fait que le dérivé indolique répond à la formule (III) suivante : 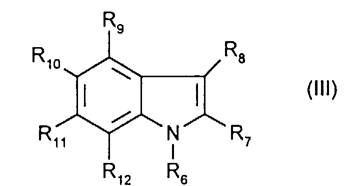 dans laquelle :
- R₆ et R₈, identiques ou différents, représentent un atome d'hydrogène ou un radical alkyle en C₁-C₄ ;
- R₇ représente un atome d'hydrogène, un radical alkyke en C₁-C₄, carboxyle ou alcoxy(C₁-C₄) carbonyle ;
- R₉ et R₁₂, identiques ou différents, représentent un atome d'hydrogène, un radical hydroxyle, alkyle en C₁-C₄, amino, alcoxy en C₁-C₄, acyl(C₂-C₄)oxy ou acyl(C₂-C₄)amino ;
- R₁₀ représente un atome d'hydrogène ou d'halogène, un radical hydroxyle, alkyke en C₁-C₄, alcoxy en C₁-C₄, amino, acyl(C₂-C₄)oxy, acyl(C₂-C₄)amino ou triméthylsilyloxy ;
- R₁₁ désigne hydrogène, un groupement hydroxy, alcoxy(C₁-C₄), amino acyl(C₂-C₄)oxy, acyl(C₂-C₄)amino, triméthylsiloxy, hydroxyalkyl(C₂-C₄)amino ;
- R₁₀ et R₁₁ peuvent former conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy; au moins l'un des groupements R₉ à R₁₂ représente un groupement OZ ou NHR, un seul au plus des groupements R₉ à R₁₂ désignant NHR ; et au plus deux des groupements R₉ à R₁₂ désignent OZ, dans le cas ou Z désigne hydrogène, ces groupements sont en position 5 et 6 ; et au moins un des groupements R₉ à R₁₂ représente hydrogène, dans le cas où un seul de ces groupements désigne hydrogène, un seul groupement parmi R₉ à R₁₂ désigne alors NHR ou OZ, et les autres groupements désignent alkyle en C₁-C₄ ; R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄ triméthylsilyle et les sels correspondants.

6. Produit indolique selon la revendication 4, caractérisé par le fait que le composé indolinique répond à la formule (IV) suivante 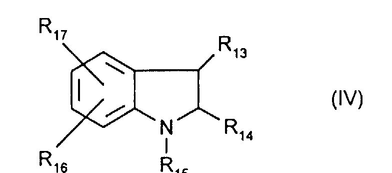 dans laquelle :
- R₁₃ et R₁₅ représentent, indépendamment l'un de l'autre, un atome d'hydrogène ou un groupement alkyle en C₁-C₄ ;
- R₁₄ représente un atome d'hydrogène, un groupement alkyle en C₁-C₄, un groupement carboxyle ou alcoxy (C₁-C₄) carbonyle ;
- R₁₆ désigne un atome d'hydrogène ou d'halogène, un groupement alkyle en C₁-C₄, hydroxyle, alcoxy (C₁-C₄), amino ou alkylamino en C₁-C₁₀ ;
- R₁₇ désigne un atome d'hydrogène, un groupement hydroxyle, alcoxy en C₁-C₄ ou amino ;
avec la condition qu'au moins un des radicaux R₄ ou R₅ désigne un groupement hydroxyle, alcoxy ou amino ; et sous réserve que lorsque R₅ désigne un groupement amino, R₄ ne peut désigner un radical alkylamino ; R₁₆ et R₁₇ peuvent également former un cycle alkylènedioxy en C₁-C₂ et sont en position 5 et 6 ; ainsi que les sels correspondants.

7. Produit indolique selon l'une quelconque des revendications 4 à 6, caractérisé par le fait que lorsque le produit est préparé par cooxydation, on utilise jusqu'à 50% en mole de dérivé indolique et/ou de dérivé indolinique par rapport au nombre total de mole de composés (I) ou (II) à oxyder.

8. Procédé de préparation des produits selon l'une quelconque des revendications 1 à 7, caractérisé par le fait que l'on procède à l'oxydation à l'air.

9. Procédé de préparation selon la revendication 8 caractérisé par le fait que l'on procède à l'oxydation à l'air en présence d'un agent alcalin et/ou d'un catalyseur métallique d'oxydation.

10. Procédé de préparation des produits selon l'une quelconque des revendications 1 à 7, caractérisé par le fait qu'il est préparé par oxydation en présence d'un agent oxydant en présence ou non d'un agent modificateur de pH et/ou d'un catalyseur métallique d'oxydation.

11. Procédé selon la revendication 10, caractérisé par le fait que l'agent oxydant est choisi parmi le peroxyde d'hydrogène, les peracides, les persels, les chlorites alcalins, l'oxyde d'argent, le chlorure ferrique, l'oxyde de plomb, le nitrite de sodium et les sels de terres rares.

12. Procédé selon la revendication 10, caractérisé par le fait que l'agent oxydant est choisi parmi les ortho- et les parabenzoquinones, les ortho- et parabenzoquinones monoimines ou diimines, les 1,2- et les 1,4-naphtoquinones, les 1,2- et les 1,4-naphtoquinones mono- ou diimines.

13. Procédé selon la revendication 11, caractérisé par le fait que l'oxydation est effectuée en utilisant dans un premier temps, soit un iodure d'un métal alcalin, alcalino-terreux ou d'ammonium, et dans un second temps, le peroxyde d'hydrogène, soit dans un premier temps le peroxyde d'hydrogène et suivi dans un second temps de l'addition d'un iodure de métal alcalin, alcalino-terreux ou d'ammonium.

14. Procédé de préparation des produits selon l'une quelconque des revendications 1 à 7 caractérisé par le fait que l'oxydation est effectuée par voie enzymatique.

15. Procédé selon l'une quelconque des revendications 10 à 14, caractérisé par le fait que l'oxydation est effectuée par introduction en solution aqueuse ou dans un milieu eau/solvant ou dans un milieu solvant pur, du ou des composés de formule (I) ou (II) avec éventuellement des indoles et/ou des indolines, et que dans un second temps, on additionne l'oxydant dans des quantités suffisantes pour former le produit indolinique.

16. Procédé selon la revendication 15, caractérisé par le fait que les solvants sont choisis parmi les alcools inférieurs en C₁-C₄, les alkylèneglycols, les alkyléthers d'alkylèneglycols, le lactate de méthyle.

17. Procédé selon l'une quelconque des revendications 8 à 16, caractérisé par le fait que les composés de formule (I) ou (II) et éventuellement les indoles et/ou les indolines représentent de 0,1 à 30% en poids par rapport au poids total du milieu réactionnel.

18. Produit indolique selon l'une quelconque des revendications 1 à 7, caractérisé par le fait qu'il est isolé sous forme de particules ayant une granulométrie moyenne inférieure à 50 microns et de préférence 20 microns.

19. Produit sous forme de poudre constituée de particules, caractérisé par le fait que les particules sont des particules minérales ou organiques, d'une dimension inférieure à 20 microns et comportant dans et/ou sur les particules un produit indolique tel que défini selon l'une quelconque des revendications 1 à 7 et 18.

20. Utilisation d'une poudre selon la revendication 19 dans et pour la préparation d'une composition cosmétique ou dermatologique.

21. Utilisation d'un produit indolique selon l'une quelconque des revendications 1 à 7 et 18 dans et pour la préparation d'une composition cosmétique ou dermatologique.

22. Composition cosmétique ou dermatologique caractérisée par le fait qu'elle contient de 0,1 à 35 % en poids dans un milieu cosmétiquement acceptable, d'un produit tel que défini dans l'une quelconque des revendications 1 à 7, 18 et 19.

23. Composition selon la revendication 22 caractérisée par le fait qu'elle se présente sous forme de lotion épaissie, de gel, de crème, de lait, de poudre, de stick, qu'elle est éventuellement conditionnée en aérosol sous forme de spray ou de mousse.

24. Composition selon la revendication 22 ou 23, caractérisée par le fait qu'elle est destinée à être utilisée pour le maquillage de la peau, des ongles, des cils et des sourcils et qu'elle se présente sous forme liquide, solide ou pâteuse, anhydre ou aqueuse.

25. Composition selon l'une quelconque des revendications 22 à 24, caractérisée par le fait qu'elle est destinée à la protection de l'épiderme humain contre les rayonnements UV et qu'elle se présente sous forme de suspension ou de dispersion dans des solvants ou des corps gras ou sous forme d'émulsion, de pommade, de gel, de bâtonnet solide et de mousse aérosol.

26. Composition selon l'une quelconque des revendications 22 à 25, caractérisée par le fait qu'elle contient des corps gras, des solvants organiques, des silicones, des épaississants, des adoucissants, des tensio-actifs, des filtres solaires, des agents anti-mousse, des agents hydratants, des parfums, des conservateurs, des agents anti-oxydants, des charges, des séquestrants, des agents de traitement, des propulseurs, des agents alcalinisants ou acidifiants ou d'autres pigments.

27. Procédé de coloration temporaire des cheveux caractérisé par le fait que l'on utilise une composition telle que définie dans l'une quelconque des revendications 22, 23 et 26.

28. Procédé de maquillage de la peau ou des phanères, caractérisé par le fait que l'on applique sur la peau ou les phanères, une composition selon l'une quelconque des revendications 22 à 26.
